# FASCICULE DE BREVET EUROPEEN

(11) **EP 3 244 819 B1**
(45) Date de publication et mention de la délivrance du brevet: **08.04.2020**
(21) Numéro de dépôt: 16700461.3
(22) Date de dépôt: 13.01.2016
(51) Int. Cl.: A61B 17/00, A61B 18/02, A61B 18/00

(54) **DISPOSITIF POUR LE TRAITEMENT COSMETIQUE DES TACHES BRUNES CUTANEES PAR CRYOGENIE**
SYSTEM ZUR KOSMETISCHEN BEHANDLUNG VON BRAUNEN HAUTFLECKEN DURCH KRYOGENESE
DEVICE FOR COSMETIC TREATMENT OF BROWN SKIN SPOTS BY CRYOGENESIS

(30) Priorité: 13.01.2015 EP 15305022
(43) Date de publication de la demande: 22.11.2017
(73) Titulaire: Cryobeauty, 28000 Chartres (FR)
(72) Inventeur: MARIN, Denis, 78620 L'Etang la Ville (FR); PACITO, Dominique, 94230 Cachan (FR)
(74) Mandataire: Alatis
(86) Numéro de dépôt international: PCT/EP2016/050565
(87) Numéro de publication internationale: WO 2016/113305

(56) Documents cités:
- US-A- 5 997 530
- US-A1- 2004 102 768
- US-A1- 2007 005 048
- US-A1- 2010 087 806
- US-B1- 6 226 996

## Description

### DOMAINE TECHNIQUE DE L'INVENTION

La présente invention concerne un dispositif pour le traitement cosmétique superficiel de taches brunes cutanées destiné à faire disparaître lesdites taches brunes.

Le dispositif vise, plus particulièrement, le traitement des taches brunes situées au niveau des mains, du visage, des membres et du décolleté de sujets atteints de telles hyperpigmentations cutanées.

La mélanogenèse par l'intermédiaire de cellules spécialisées appelées mélanocytes, à l'origine de la coloration de la peau, est influencée par des facteurs extérieurs qui entraînent une augmentation de la production de mélanine et, par voie de conséquence, une coloration localisée plus importante. Il y a alors formation de taches pigmentaires.

Les taches brunes (lentigos, dermatites actiniques) peuvent apparaître à partir de 30 ans, parfois dès l'adolescence, et sont localisées principalement sur les mains, le visage et le décolleté. Elles sont notamment la conséquence de l'exposition trop importante au soleil. L'héliodermie désigne l'ensemble des conséquences du soleil sur la peau, tel que les taches, le relâchement de la peau, l'apparition de rides, avec un aspect fripé, de peau sèche. Elle peut se manifester au niveau des taches par :
- éphélides ou « taches de rousseur » dont le nombre et l'intensité augmentent sous l'effet de l'exposition aux ultraviolets ;
- lentigos solaires des zones surexposées au soleil ;
- hyperchromie (pigmentation anormale) de type mélasma dont l'intensité est directement liée à l'exposition aux ultraviolets A et B ;
- « dermite en breloque » qui est une séquelle d'une photosensibilisation induite par un parfum.

Le vieillissement cutané intrinsèque entraine une atrophie qui s'accompagne de phénomènes de relâchement cutané, de dessèchement et de troubles pigmentaires. Le principal trouble pigmentaire est le lentigo sénile, qui apparaît vers la cinquantaine sur les parties découvertes de la peau, et plus particulièrement sur le dos des mains et parfois sur le visage. Petites taches de couleur brune, lisses, sans relief, de quelques millimètres à quelques centimètres de diamètre, leur pronostic est toujours bénin. Ces taches peuvent également se développer chez le sujet âgé, lors d'inflammations ou de dérèglements hormonaux.

Elles sont considérées comme une disgrâce esthétique plus ou moins importante selon l'intensité de l'hyperpigmentation. La cause originelle est mal connue. Les conséquences physiologiques sont mieux étudiées : (1) augmentation de la synthèse de mélanine, (2) accélération du transfert de la mélanine des mélanosomes vers les kératinocytes et enfin (3) migration plus rapide des mélanocytes vers la surface cutanée.

### ETAT DE LA TECHNIQUE ANTERIEURE

Deux axes de traitement sont possibles : l'atténuation de l'hyperpigmentation ou la destruction de la mélanine ou des tissus hyperpigmentés.

L'atténuation de l'hyperpigmentation peut être réalisée par des agents dépigmentants ayant une action pharmacologique, qui agissent sur les conséquences physiologiques. On peut citer à titre d'exemples, le monométhyléther d'hydroquinone, le méquinol, la trétinoïne, ou encore l'acide kojique. La durée du traitement est longue et ses modalités d'application sont contraignantes (plusieurs applications par jour). La compliance est donc faible. De plus, le risque de récidive est très important puisque la cause originelle n'est pas traitée. Le patient peut réaliser cette opération à son domicile, l'assistance du dermatologue n'étant pas nécessaire. Les effets secondaires et indésirables sont principalement des réactions inflammatoires localisées, des réactions allergiques aux principes actifs et une sensation de brûlure pour certains actifs.

Les dermocorticoïdes ont un potentiel dépigmentant certain, surtout lorsqu'ils sont appliqués sous pansement occlusif. Ils sont utilisés dans des crèmes à base d'hydroquinone, mais c'est davantage pour diminuer l'irritation induite par la préparation que pour en accroître l'efficacité. Le monobenzyléther d'hydroquinone est à proscrire. En effet, très puissant et peu maniable, il donne de fréquentes dépigmentations à distance de la zone traitée. Il a été par le passé responsable d'accidents cosmétiques sévères, avec des leuco-mélanodermies définitives. Certains praticiens l'utilisent encore dans le traitement des vitiligos étendus pour compléter la dépigmentation des zones de peau saine mais cela nécessite deux applications par jour pendant plusieurs mois, à une concentration de 5 à 20%. En outre, ce produit peut s'avérer irritant ou allergisant.

La destruction de la mélanine ou plus généralement des tissus hyperpigmentés est réalisée par la technique de cryothérapie chez le dermatologue, c'est-à-dire l'application sur la surface à traiter de froid, par un professionnel de santé. Cette technique professionnelle classique et ancienne ne permet ni la maîtrise du niveau, ni de la durée d'exposition à la température obtenue.

En effet, le dermatologue utilise une bonbonne pulvérisant des gaz qui ont généralement des niveaux de température très bas comme l'azote liquide à - 196°C, sans pouvoir contrôler de manière précise le débit et la durée de pulvérisation puisqu'il actionne mécaniquement lui-même, l'ouverture de la bonbonne de gaz cryogénique. Par convention, les médecins ont défini des durées d'application du froid selon la nature du problème à traiter, mais sans aucune autre précision.

Ainsi, l'azote liquide doit être appliqué sur une verrue vulgaire en une seule fois en comptant 10 secondes, sur une verrue plantaire entre 20 et 30 secondes et en deux fois et sur un lentigo solaire pendant 5 secondes en une seule fois. Le manque de précision du contrôle tant pour la gestion du temps d'application du froid que pour la détermination du niveau des très basses températures à appliquer, entraîne de grandes disparités d'un traitement à l'autre, une absence totale de reproductibilité et donc au final, des variations importantes sur l'efficacité obtenue.

De plus, l'utilisation de l'azote liquide (-196°C) provoque une brûlure du second degré entraînant une nécrose de l'ensemble des populations de cellules du tissu traité, sans différenciation, qui se traduit par des cicatrices résiduelles, des risques accrus d'hypo-pigmentation et une douleur à l'application.

Les autres techniques sont le peeling et la micro-dermabrasion qui génèrent une action superficielle d'atténuation de la couleur de la tache brune sans l'éliminer, et enfin les lasers qui présentent des inconvénients similaires à ceux de la cryothérapie.

La cryothérapie conventionnelle est utilisée sur certaines lésions épidermiques, comme les lentigos actiniques. La technique est très variable suivant le matériel utilisé pour appliquer le froid. Les dermatologues se servent essentiellement de sprays pulvérisant de l'azote liquide sur la surface à détruire. Les sprays peuvent être ouverts ou fermés, l'azote étant alors projeté dans des cônes de néoprène dont la taille est adaptée à la surface à traiter. Cette méthode est souvent précédée d'un curetage de la zone à traiter qui permet de réaliser un prélèvement pour examen anatomopathologique et de déterminer de façon plus précise les limites de l'extension afin d'optimiser les résultats. Un ou plusieurs cycles de congélation - décongélation sont ainsi réalisés et, en règle générale, deux cycles sont effectués.

Une autre méthode consiste à appliquer l'azote liquide par le moyen de cryodes fermées dont la taille est adaptée à la surface cible. Un contrôle de la température intratissulaire à l'aide d'aiguilles, par thermocouple ou par impédancemétrie, est possible mais n'est pas réalisé systématiquement. L'existence d'un halo de congélation en périphérie de la zone cible et le temps de congélation et de décongélation sont utilisés pour apprécier la destruction tissulaire. Il s'agit d'une technique relativement simple, ambulatoire, qui permet de traiter des lésions multiples et qui n'est pas contre-indiquée chez les malades sous anticoagulants. L'évolution de la cryonécrose se fait sur plusieurs semaines avec la nécessité de pansements à renouveler. Elle peut donner lieu à des séquelles dyschromiques hypo- ou hyperpigmentées.

Lorsque les taches cutanées sont très nombreuses ou très larges, un traitement à l'azote liquide s'avère trop violent et on peut préférer utiliser par exemple de la neige carbonique ou N20.

Le brevet US 7, 963,959 B2 décrit un dispositif automatisé piloté à l'aide d'un système d'acquisition d'images pour le traitement de nombreuses zones cutanées au moyen de divers fluides permettant une action de cryothérapie, dont des CFC. Ce dispositif est destiné à être mis en œuvre dans un cadre médicalisé.

Les demandes de brevet FR 2 885 059 et FR 2 885 539 décrivent des dispositifs manuels pour appliquer un fluide cryogénique contenu dans une réserve d'aérosol sur une zone cutanée à traiter, via un gicleur et une buse d'éjection, la durée d'application étant contrôlée par une minuterie mécanique. Ces dispositifs sont conçus pour permettre un traitement en dehors d'un milieu hospitalier ou médicalisé. Toutefois les gestions du débit de fluide et de la durée d'application du fluide se sont avérées en pratique non maitrisées, très peu fiables et non reproductibles. En effet, la structure même du dispositif (grand nombre de composants), les jeux et tolérances mécaniques et thermiques des différentes pièces et la manière dont il est conçu fait que les applications de fluide cryogénique sont difficilement reproductibles. En effet, ces applications donnaient lieu à des variations importantes de températures locales instantanées des zones traitées d'un essai à l'autre du fait de cette absence de reproductibilité, et ne présentaient pas la sécurité et l'efficacité attendues avec ce type de dispositif à cause des risques de brulures importantes et de nécrose étendue.

Par ailleurs la mise en oeuvre des dispositifs de l'art antérieur produit un effet de lyse cellulaire non sélective, c'est à dire qu'ils produisent une nécrose de l'ensemble des populations cellulaires des tissus des zones traitées, et ceci de manière indifférenciées sur toutes les populations de cellules (mélanocytes, kératinocytes, fibroblastes...). En effet, leur nature même ou leurs mécanismes de fonctionnement et de mise en œuvre ne permettent pas une gestion ni un contrôle précis de la dose délivrée et de la durée d'application du fluide cryogénique sur une zone déterminée ni, par conséquent, du niveau de la température souhaitée sur l'ensemble de la zone traitée. La plage des températures effectivement appliquées dans les tissus étant large, cette méthode ne permet pas d'obtenir une action cryo-cyto-sélective vis-à-vis des populations cellulaires présentes. On appelle ainsi action cyto-sélective la possibilité d'agir spécifiquement sur une population donnée de cellules (par exemple uniquement les mélanocytes), sans agir sur les autres populations de cellules. Ainsi, les dispositifs de l'art antérieur ne permettent pas d'agir uniquement sur une population de cellules (par exemple les mélanocytes). Pour ce faire, il faudrait contrôler très précisément la température appliquée sur les cellules, et ceci non seulement au niveau de la température la plus basse atteinte mais également au niveau de la cinétique de la variation de température. En effet, certaines populations de cellules réagissant différemment au froid par rapport à d'autres, en appliquant du froid dans une certaine plage de température et selon une certaine cinétique, il est possible alors d'agir spécifiquement sur une population de cellules donnée en provoquant leur lyse, sans affecter les autres populations. Le fait d'appliquer du froid afin d'obtenir un effet cyto-sélectif est appelé action cryo-cyto-sélective ou cryo-cyto-sélectivité.

Jusqu'à présent, l'homme du métier ne cherchait qu'une action de nécrose cellulaire locale indifférenciée car il ne travaillait que par convention de durée de temps, ne connaissant pas la cryo-cyto-sélectivité. En conséquence, l'homme du métier n'avait pas pensé et ne pouvait pas imaginer de moyens permettant d'exercer une action cryogénique cyto-sélective pour un soin cosmétique. De plus, les appareils à sa disposition n'étaient pas d'utilisation ambulatoire, ni simples ni rapides à mettre en œuvre ou permettant un réglage automatisé très précis de la température optimale qui soit suffisante pour atteindre une zone cible.

Il s'avère, en outre, que les dispositifs cryogéniques traditionnels connus rencontrent des problèmes de givrage et de colmatage du fait de la congélation brusque de la vapeur d'eau présente dans l'environnement immédiat du gicleur, lors de la détente du fluide cryogénique. Ces problèmes sont accentués par la nature des matériaux non hydrophobes utilisés jusqu'à présent pour la réalisation du gicleur.

Ce phénomène est d'autant plus problématique que le gicleur dont le diamètre doit être très faible sur une longueur importante est généralement réalisé en métal, pour des raisons de résistance mécanique.

Par conséquent, le gicleur des dispositifs cryogéniques actuels permettant la temporisation du débit du fluide est le siège de phénomènes physiques de rétention et de conduction du froid en amont qui perturbent le bon fonctionnement des moyens de temporisation, qu'ils soient mécaniques (ressorts, cames,...) ou qu'ils soient électroniques (électrovanne,....) car ils sont tous très sensibles aux basses températures.

De plus, l'homme du métier est confronté à des problématiques techniques de régulation de la puissance et d'importance du débit du fluide réfrigérant. En effet, lorsqu'il utilise un container pressurisé, il doit actionner un levier permettant d'ouvrir ou de fermer une valve qui conditionne la sortie du fluide réfrigérant. Le temps d'application est donc extrêmement variable d'une pulvérisation à l'autre et par conséquence la dose de froid délivrée ne peut être maîtrisée. Enfin, une vis sur l'embout permettant de modifier le débit ne permet pas en cas de modification de retrouver une position de débit antérieure.

Ces problèmes ont un impact très significatif sur la diffusion du fluide cryogénique, et par conséquent sur la cinétique de température obtenue sur les tissus traités. Il n'est donc pas possible aujourd'hui, avec les dispositifs existants, de produire des effets uniquement cosmétiques par cryogénie cyto-sélective.

### EXPOSE DE L'INVENTION

Au regard des techniques et produits utilisés actuellement, l'invention vise donc à résoudre les problèmes techniques posés par l'art antérieur en proposant un dispositif pour :
- Eliminer totalement les taches brunes et non pas atténuer leur couleur,
- Obtenir un effet immédiat,
- Eliminer les taches brunes en une seule application;
- Obtenir le résultat attendu sans provoquer de marques sur la peau, sans générer de douleur et d'apparition de rougeurs pendant et après l'application du froid,
- Supprimer tous risques de givrage et/ou de conduction du froid par le gicleur pour éviter toute perturbation du système de temporisation électronique ou mécanique du dispositif cryogénique,
- Limiter et maitriser le débit du fluide réfrigérant,
- Maîtriser parfaitement le niveau de température à atteindre et à maintenir en surface cutanée pendant un temps donné (cinétique de la température),
- Permettre un usage simple et rapide du dispositif.

Dans cet objectif, l'invention propose un dispositif selon la revendication 1 destiné au traitement cosmétique des taches brunes cutanées.

Le traitement utilisant le dispositif selon l'invention engendre une action cyto-sélective grâce à une cryogénie appropriée. Dans le cadre de la présente divulgation, on entend par action de cryogénie cyto-sélective ou cryo-cyto-sélectivité le fait d'agir de façon sélective au moyen d'un agent fluide réfrigérant sur une population de cellules d'un tissu considéré, sans agir sur les autres populations de cellules. Le procédé de traitement cosmétique selon la présente divulgation agit uniquement par un niveau de froid maitrisé, sur les mélanocytes situés entre la couche cornée et la couche basale (jonction dermo-épidermique), dans l'épiderme. Avec la couche cornée, l'épiderme est la partie la plus superficielle de la peau. Ainsi, cette action superficielle s'effectue sans détruire les kératinocytes.

Des modes d'exécution du dispositif de l'invention sont définis par les revendications dépendantes.

### BREVE DESCRIPTION DES FIGURES

D'autres caractéristiques et avantages de l'invention ressortiront à la lecture de la description qui suit, en référence aux figures annexées, qui illustrent :
- la figure 1 est une vue schématique en coupe longitudinale d'un mode de réalisation du dispositif de cryogénie cyto-sélective selon l'invention ;
- la figure 2 est une courbe expérimentale montrant l'effet thermique obtenu à l'aide d'un dispositif selon la figure 1.
- la figure 3 est une vue en coupe longitudinale d'une variante de réalisation du dispositif de cryogénie cyto-sélective selon l'invention ;
- les figures 4A, 4B et 4C représentent des vues de détail en coupe de trois variantes de gicleurs perfectionnés susceptibles d'être utilisés dans le dispositif.
- La figure 5 représente une vue de détail en coupe longitudinale d'une variante de gicleur utilisée dans le dispositif de la figure 3.

### DESCRIPTION DETAILLEE DES MODES DE REALISATION

La cryothérapie est une méthode qui est perçue comme douloureuse chez 64% des patients traités lorsque l'application du froid est d'une durée supérieure à 10 secondes. Dans le cas où l'application du froid se fait pendant une période inférieure à 10 secondes, la douleur n'est plus perçue que par 44% des patients. Plus le temps d'application est faible, moins forte est la douleur. Cependant, une faible exposition au froid diminue considérablement l'efficacité du traitement puisque dans ce cas, seulement environ 30% des patients sont guéris. Il apparaît clairement que le temps d'application du froid à une conséquence directe sur l'efficacité du traitement et l'intensité de la douleur ressentie.

La réfrigération d'un tissu conduit à des changements d'état physique et, selon les conditions d'application du froid, à sa préservation ou au contraire à son altération. Le choc thermique appliqué dans le cadre de la présente divulgation est un très grand abaissement de la température en un minimum de temps. Le procédé est mis en oeuvre par une congélation très rapide, suivie d'un réchauffement lent durant lequel l'action du froid va se prolonger. L'abaissement très brutal de la température engendre, avant même que le tissu soit solidifié, une micro-cristallisation de l'eau intracellulaire ; ces microcristaux induisent altérations membranaires, dénaturation des protéines de structure et enzymatiques, surconcentrations ioniques, toutes conditions conduisant à un effet délétère sur les cellules. Une recristallisation de l'eau en excès pendant la phase de réchauffement majore encore la lyse cellulaire. Dans les conditions normales la température cutanée est aux alentours de 34°C. C'est cette température qui doit être abaissée au maximum en un minimum de temps.

Les mélanocytes sont situés au niveau de la jonction dermo-épidermique et migrent au cours des quatre stades de leur maturité vers la surface de la peau, c'est à dire vers la couche superficielle de l'épiderme. Ces mélanocytes contiennent des mélanosomes, qui sont des vacuoles contenant la mélanine. La maturation des mélanosomes et la concentration en mélanine se fait à l'intérieur des mélanocytes. Puis les mélanosomes sont transférés au niveau des dendrites des mélanocytes et vers les kératinocytes, qui les intègrent dans leurs structures cellulaires. Ils se placent au dessus du noyau pour le protéger des rayonnements UV. Puis les mélanosomes sont dégradés par des enzymes. La mélanine libérée est éliminée au niveau de la surface épidermique par la desquamation de la couche cornée et dans le derme par voie lymphatique.

Les hyperpigmentations résultent d'un trouble de la mélanogenèse, avec activité accrue au niveau des mélanosomes et parfois d'un transfert plus important du pigment dans les kératinocytes de la couche de Malpighi, ou d'une accumulation de mélanine dans le derme. Il s'agit donc d'une hypermélanocytose, située au niveau de la couche basale : l'hypermélanocytose se caractérise par l'augmentation du nombre de mélanocytes, ou une augmentation de la synthèse de mélanine par les mélanocytes. La cellule clé et, par voie de conséquence, la cellule cible, est donc le mélanocyte. Cependant, les kératinocytes ne doivent pas être touchés car ils constituent la protection du tissu épidermique contre le rayonnement UV.

L'épaisseur de l'épiderme varie en fonction de la zone concernée, de 0,02 mm au niveau de la peau du visage à 5 mm au niveau de la plante des pieds. Son épaisseur moyenne est de 0,1 mm. Au niveau des mains (dessus des mains), les mélanocytes sont situés à 0,1 mm de la surface de la peau.

Dans un épiderme soumis au froid, les mélanocytes restent viables s'ils sont soumis à une température comprise entre 0 et - 4°C.
Entre - 4°C et - 7°C, on observe une lyse des granules contenant les pigments, c'est-à-dire les mélanosomes contenant la mélanine, et par la suite un début de digestion enzymatique de la mélanine dans les mélanocytes et dans les kératinocytes dans la couche profonde de l'épiderme, près de la couche basale. Entre - 7°C et - 30°C, on observe une disparition des mélanocytes et, en dessous, il n'y a pas de réapparition de mélanocytes (troubles de dépigmentation). La destruction des kératinocytes intervient pour des températures inférieures à - 20°C, avec une destruction très importante des populations de mélanocytes fortement différenciés.

L'efficacité du traitement cosmétique de l'hyperpigmentation due aux mélanocytes, sans dommages importants pour les kératinocytes, se situe dans une plage comprise entre - 4°C et - 15°C et de préférence entre - 5°C et - 12°C. Le froid délivré à la surface de la peau doit y générer une température comprise de préférence entre - 5°C et - 12°C pendant 2 à 10 secondes, pour agir sur les mélanocytes et la mélanine de manière cyto-sélective. Ainsi, l'application d'un fluide à une température comprise entre - 5°C et - 12°C pendant une durée de 2 à 10 secondes, permet d'agir sur la mélanine et les mélanocytes, sans endommager les kératinocytes, selon un principe de cryo-cyto-sélectivité, c'est à dire une sélectivité cellulaire par la cryogénie. La balance bénéfice (action sur la mélanine et le mélanocyte entre - 5°C et - 12°C) versus risque (destruction du kératinocytes en dessous de - 20° C) est donc importante et va dans le sens d'une grande sécurité d'utilisation du traitement cosmétique et d'une absence d'effets secondaires (douleurs et cicatrices).

Certains fluides réfrigérants ont des propriétés de condensation d'eau plus marquées que d'autres. C'est le cas notamment du diméthyl-éther, fluide cryogénique utilisé dans certains produits pour traiter des verrues. Lorsque ce mélange arrive dans un embout en mousse, il y a condensation de la vapeur d'eau contenue dans l'air du fait du contact de la mousse froide avec l'air ambiant. On peut observer systématiquement la formation de gouttelettes qui gèlent aussitôt sur la surface de l'embout lorsque celui-ci est maintenu à l'air libre. Ce fluide demeure longtemps à l'état liquide sur la peau, il s'évapore lentement, emprisonné dans l'embout. Lors de l'application de cet embout sur la peau, il y a ainsi une certaine quantité d'eau qui se trouve déposée sur la zone traitée et cette humidité renforce très nettement la sensation de douleur. Par contre, le difluoroéthane, code 152A, s'évapore très vite, ce qui permet au froid de pénétrer plus rapidement dans la peau. Cette vitesse d'évaporation et une pulvérisation directe sans contact sur la peau évitent la condensation d'eau et son "emprisonnement". Le difluoroéthane (152A) a été choisi pour son Point d'ébullition à -25°C et sa pression de vapeur de 5,3 bars à 20°C, et sa faible chaleur latente d'évaporation (160 KJ/kg), afin de produire un fluide très volatile pour éviter la formation des gouttes sur la peau, et, par voie de conséquence, la sensation de douleur tout en apportant suffisamment de froid pour être efficace.

Lorsque la peau est soumise à une source de froid appliquée à sa surface, elle subit un abaissement rapide de sa température. En l'espèce l'application d'un jet de difluoroéthane sans contact direct avec la peau produit une variation de la température superficielle de l'ordre de 10 à 20°C par seconde. Il permet donc de diminuer la température du tissu d'environ 34°C jusqu'à - 5°C à - 12°C, soit un différentiel d'environ 40°C, en un temps d'un peu plus de 2 secondes, et, dans la pratique, de 2,5 secondes à 3 secondes, du fait des pertes thermiques.
Une variante de réalisation utilise du gaz 134A, c'est-à-dire le tétrafluoroéthane.

Le changement de température progresse à raison de 0,5 à 1mm par seconde à travers les couches de la peau.
En conséquence, la température de la couche basale engendrée par cette source de froid devient égale à la température à la surface de la peau dans un intervalle de temps d'environ 0,2 secondes.
Dans ces conditions, en moins de 1 seconde, la température des mélanocytes au niveau de la couche basale sera identique à celle à la surface cutanée. Un temps de pulvérisation du fluide cryogénique de l'ordre de 3 secondes est donc optimal en vue de l'effet recherché.

Selon un mode d'exécution préféré, le procédé est mis en œuvre à l'aide d'un diffuseur directif de difluoroéthane (152A) avec système de temporisation automatisé, préréglé à trois secondes, dont les principaux éléments constitutifs sont décrits ci-dessous.

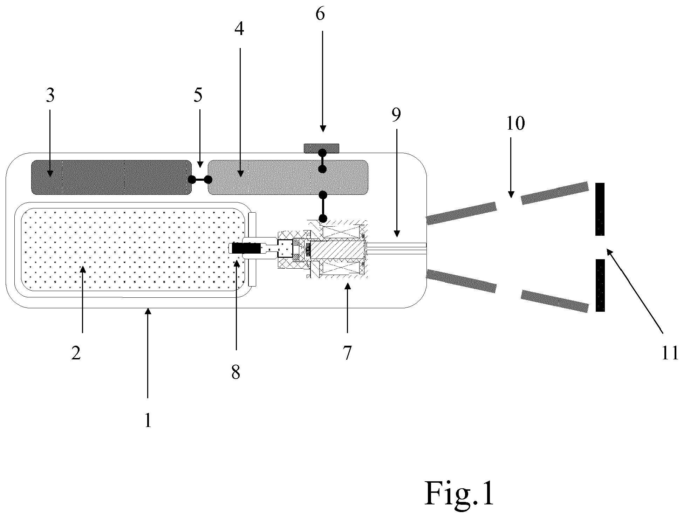

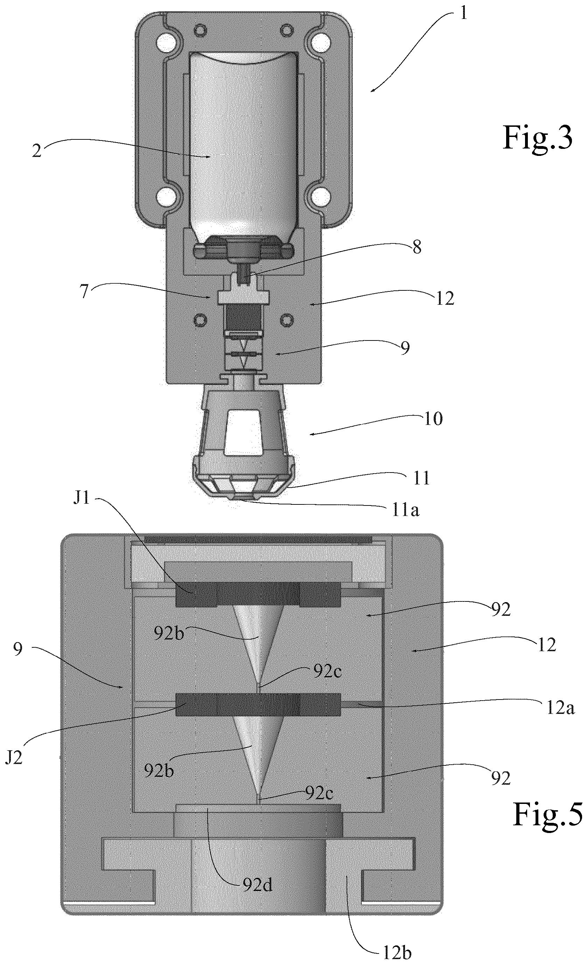

Comme le montrent les figures 1 et 3, les composants du dispositif sont entourés d'un carter 1. Ce carter contient une cartouche 2 de fluide cryogénique, de préférence du 152A, éventuellement du 134A (tétrafluoroéthane) sous une pression d'environ 6 bars et au maximum de 10 bars. La cartouche 2 est reliée à une électrovanne 7 par l'intermédiaire d'un stem 8 permettant l'échappement du fluide de l'intérieur de la cartouche vers l'extérieur. En position de repos du dispositif, le stem 8 laisse pénétrer le fluide dans la chambre amont de l'électrovanne 7. Le carter 1 contient une source d'énergie, par exemple sous forme de pile(s) électrique(s) 3. La source d'énergie est reliée via un contacteur 5 à un système électronique de temporisation 4 permettant le passage de fluide dans l'électrovanne 7 pendant un temps prédéterminé. Le système électronique de temporisation 4, et par voie de conséquence l'électrovanne 7, est actionné par un bouton de déclenchement 6. En aval de l'électrovanne 7 est agencé un gicleur 9 représenté schématiquement selon deux variantes sur les figures 1 et 3. Cet ensemble permet le prélèvement d'une dose précise et reproductible de fluide cryogénique et son éjection via le gicleur pendant une durée prédéterminée, par exemple trois secondes, avec une précision de 0,1 seconde. La précision de la temporisation de l'électrovanne est nécessaire pour garantir une action cryo-cyto-sélective qui permet d'éviter d'atteindre des températures délétères pour les kératinocytes.

De préférence, l'extrémité du stem 8 qui est engagée à l'intérieur de la cartouche 2, est pourvue d'une douille coaxiale (non représentée) dans laquelle sont ménagées des fentes périphériques longitudinales permettant le passage du fluide cryogénique sous pression.

Ces fentes sont destinées à ne laisser passer le fluide que lorsque le dispositif est orienté verticalement avec la cartouche 2 en position haute (tête en bas).

Cette configuration permet d'interdire l'utilisation du dispositif dans d'autres positions pour des raisons de sécurité. Elle permet également d'optimiser la facilité de manipulation du dispositif sur les taches brunes de la main par une diffusion de haut en bas et avec la seule autre main, sans l'aide d'une tierce personne.

Le gicleur 9 débouche dans une buse 10 agencée à l'extérieur du carter 1. La buse 10, qui est conique dans le mode d'exécution représenté sur la figure 1, se termine par un embout 11 avec un orifice permettant le contact entre le jet de fluide froid (à l'état gazeux) et une zone cible de la peau. Cet orifice peut présenter une aire qui correspond au diamètre des lentigos les plus étendus que l'on puisse traiter dans le cadre d'un procédé cosmétique sans risque de confusion avec un éventuel mélanome. L'orifice peut donc présenter par exemple une forme circulaire avec un diamètre de 6 mm. De la sorte, une tache cutanée peut être traitée en une seule application. La forme conique de la buse, sa longueur de l'ordre de 35 mm, ses ouvertures latérales et son embout ont été conçus pour focaliser la diffusion du fluide sur une zone précise des tissus

Dans le mode de réalisation de la figure 1 le fluide cryogénique passe successivement de la cartouche 2 et du stem 8, dont le diamètre de sortie peut varier de 3 à 4 mm, dans l'électrovanne 7, dont les diamètres d'entrée et de sortie peuvent varier de 0,15 mm à 0,25 mm.
Le fluide cryogénique passe dans une chambre de l'électrovanne 7 qu'il suit sur une longueur pouvant être comprise entre 10 et 30 mm.
A la sortie de cette électrovanne, il pénètre par un orifice dans le gicleur 9, dans lequel la longueur de parcours du fluide est comprise entre 3 et 12 mm avec un diamètre intérieur de 0,15 à 3,5 mm et qui peut être composé de l'assemblage d'une ou plusieurs pièces identiques en matériau hydrophobe et sans ou à très faible conductivité thermique.
Ce gicleur est constitué d'un élément qui est long et particulièrement étroit et dans lequel circule le fluide avant son éjection et sa détente à la pression atmosphérique sur ou au voisinage immédiat de la zone cutanée à traiter. C'est précisément cette détente qui est un phénomène endothermique produisant l'effet cryogénique.
Le gicleur contribue à réduire la vitesse initiale du fluide cryogénique et à favoriser la projection du liquide froid vaporisé sur une zone cutanée dans les conditions appropriées de température et de durée imposées par le soin cosmétique apporté par le dispositif selon l'invention.

Dans le cadre de la présente divulgation, il a été constaté que des modifications du diamètre, de la longueur et de la forme du gicleur 9 permettent d'influencer considérablement le débit du gaz et que ces modifications jouent un rôle important, en combinaison avec des ajustements du temps d'ouverture de l'électrovanne 7, sur la température de la surface de la zone de peau atteinte.

En particulier, et selon un mode d'exécution particulièrement préféré, un gicleur dont la longueur de parcours du fluide est comprise entre 3 et 12 mm pour un diamètre intérieur de passage de 0,15 à 3,5 mm, en combinaison avec un temps d'ouverture de l'électrovanne de trois secondes, permet d'obtenir une plage de température conduisant à une action cryo-cyto-sélective efficace sur la zone traitée des tissus.

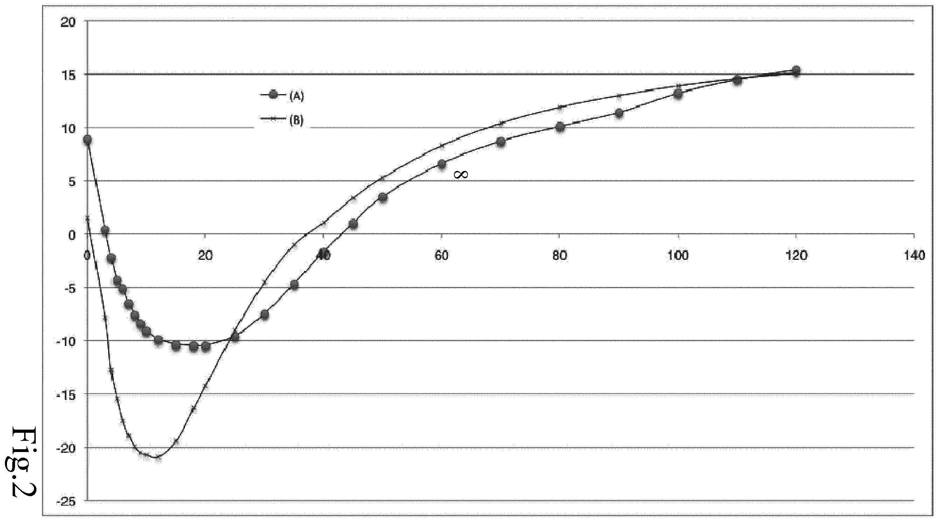

La figure 2 illustre ce qui précède en montrant la température de surface de la peau obtenue après trois secondes d'ouverture de l'électrovanne avec (A) et sans (B) le gicleur 9. L'absence de gicleur ne permettrait pas d'atteindre la plage de température permettant une action de cryo-cyto-sélectivité puisqu'on atteindrait une température délétère pour les kératinocytes.

En outre, en vue d'éviter, d'une part, les phénomènes de givrage et de protéger les moyens de délivrance du fluide et, notamment, d'isoler les composants électroniques ou mécaniques du système de temporisation de l'électrovanne vis à vis des basses températures et de limiter, d'autre part, la puissance du débit, l'invention définit un gicleur spécifique permettant d'augmenter la résistance à l'écoulement du fluide cryogénique en aval de l'électrovanne 7.

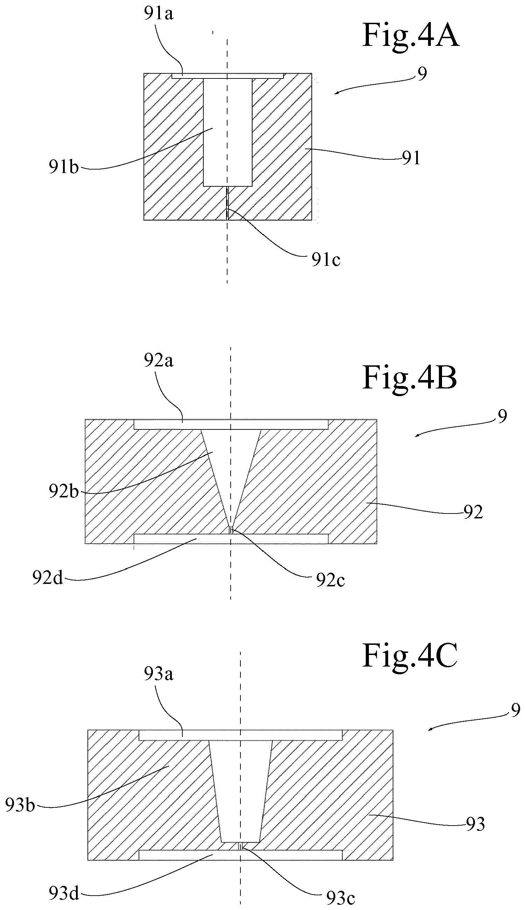

Les figures 4A à 4C représentent des variantes d'un gicleur et en particulier la figure 4b représente un gicleur comportant une conduit interne axial et cylindro-conique.

Le gicleur 9 illustré par la figure 4A comprend un corps cylindrique 91 monobloc sur la face supérieure duquel est ménagée une cavité circulaire amont 91a.
Le corps 91 a ici une hauteur comprise entre 4 et 12 mm et, de préférence, une hauteur de 10,50 mm, un diamètre extérieur d'environ 12 mm tandis que la cavité 91a présente une faible profondeur (de l'ordre de 0,40 mm) et un diamètre intérieur d'environ 8 mm.
Cette cavité est destinée à recevoir un joint d'étanchéité annulaire (tel que J1 figure 5) dont l'épaisseur correspond sensiblement à la profondeur de la cavité 91a.
La cavité 91a se prolonge vers l'aval et à l'intérieur du corps par un conduit axial également cylindrique 91b de passage du fluide dont le diamètre intérieur est ici de 3,5 mm et dont la longueur est ici comprise entre 8 et 9 mm.
Compte tenu de ses dimensions relatives, le conduit 91b forme une chambre de rétention et d'accumulation transitoire du fluide qui assure, par voie de conséquence, le ralentissement de son débit. Le fluide est ensuite éjecté vers le bas et à l'extérieur en direction de la zone cutanée à traiter, successivement, au travers d'un canal axial 91c de très faible diamètre (entre 0,15 et 0,25 mm) par rapport à celle du conduit 91b puis de la buse 10 et dont la longueur est, de préférence, de 0,3 à 2,4 mm.
Dans le conduit 91b, le jet de fluide sortant de l'électrovanne 7 est au moins partiellement liquide car la détente n'y est encore que partielle et il se trouve soumis à des turbulences résultant de l'impact du jet de fluide libéré et pulvérisé à partir de l'électrovanne 7 contre les parois du conduit. Ce régime de turbulences contribue également au ralentissement du débit de fluide.

Une autre variante du gicleur de l'invention est illustrée par la figure 4B en relation avec la figure 5.
La cavité 92a est, comme la cavité 91a, destinée à recevoir un joint d'étanchéité annulaire (voir J1, J2 figure 5) dont l'épaisseur correspond sensiblement à la profondeur de cette cavité.
Toutefois, à la différence du conduit 91b de la figure 4A, le conduit axial 92b est ici de profil conique avec un diamètre d'entrée en amont entre 2 et 3 mm pour une longueur comprise entre 3 et 5 mm.
Le conduit 92b se prolonge par un canal axial 92c de très faible diamètre (entre 0,15 et 0,25 mm) et de courte longueur (entre 0,3 et 2,4 mm et, de préférence, de 0,5 mm) qui débouche à l'extérieur vers le bas au centre d'une cavité inférieure coaxiale 92d identique à la cavité supérieure 92a et destinée à recevoir également un joint d'étanchéité annulaire (voir figure 5).
La figure 4C représente encore une autre variante du gicleur 9 dans laquelle le conduit axial interne 93b est ici tronconique avec un diamètre d'entrée en amont compris entre 2,0 et 3,0 mm et un diamètre intérieur en partie basse compris entre 1,0 et 2,0 mm.
Le conduit 93b débouche dans la cavité inférieure 93d via un canal 93c identique au canal 92c de la figure 4B.

Pour assurer un meilleur isolement thermique de l'électrovanne 7 et, plus particulièrement, du système électronique ou mécanique assurant la temporisation, vis à vis des basses températures du fluide pénétrant immédiatement en aval dans le gicleur 9, il est prévu de réaliser le corps du gicleur avec un matériau hydrophobe et sans ou à très faible conductivité thermique tel que du PTFE, PFA, POM ou un mélange POM + PTFE. En effet, ces matériaux en ne retenant pas les gouttes d'eau (ni par absorption, ni par adsorption), permettent ainsi d'écarter les risques de givrage du gicleur.
Par ailleurs, ces matériaux étant thermiquement isolants, permettent de protéger le système de temporisation, qu'il soit mécanique ou électronique, en évitant des dysfonctionnements.
Avantageusement, le corps de gicleur pourra être réalisé par moulage de cette matière plastique hydrophobe et non conductrice thermiquement.

La figure 5 représente une variante du gicleur du dispositif de l'invention réalisée par l'assemblage et le raccordement en série de deux corps 92 identiques tels que représentés sur la figure 4B.
Il serait toutefois possible, sans sortir du cadre de la présente divulgation, de prévoir l'assemblage et le raccordement fluidique de deux corps de dimensions et profils différents, notamment, selon des variantes illustrées par les figures 4A à 4C.
Chacun des deux corps 92, tel qu'illustré par la figure 4B, est cylindrique et présente une cavité circulaire amont 92a analogue à la cavité 91a de la figure 4A dans laquelle vient se loger au moins un joint d'étanchéité annulaire J1, J2.
Les deux corps 92 ont chacun un diamètre d'environ 12 mm comme le corps 91 de la figure 4A et leur hauteur respective est comprise entre 4,5 et 5,5 mm.
Les deux corps 92 sont montés l'un contre l'autre de façon empilée et coaxiale en écrasant les joints J, respectivement, supérieur J1 et intercalaire J2, à l'intérieur du compartiment amont 12a d'un boîtier 12 dont les dimensions sont conçues à cet effet.
Le raccordement en série des conduits 92b, 92c d'au moins deux corps 92 de gicleur 9 en aval de l'électrovanne 7 permet de limiter et/ou de ralentir le débit du fluide cryogénique en évitant ainsi tout à la fois une détente trop brutale qui serait susceptible de provoquer le givrage et une température trop basse sur la zone d'application cutanée.
Selon un mode de réalisation préféré, le conduit 92c a un diamètre de 0,17 mm sur une hauteur comprise entre 0,5 et 0,9 mm et de préférence 0,5 mm.

De préférence, le boîtier 12 sera réalisé d'une seule pièce avec le carter 1 (figures 1 et 3) et est raccordé, via un compartiment aval 12b réalisé en partie basse, à la buse 10 communiquant avec l'embout 11 formant collimateur.

Les figures 3 et 5 illustrent un mode de raccordement de la buse 10 et de l'embout 11 au boîtier 12 du gicleur 9.
Selon une variante, il serait possible de prévoir un raccordement amovible (par exemple, par baïonnette) et/ou réglable en hauteur (par exemple, par vissage) de la buse 10 sur le boîtier 12 de façon à ajuster la position de l'embout et la concentration en fluide cryogénique sur la zone cible à traiter.

Dans la variante représentée sur la figure 3, l'embout 11 est réalisé sous forme d'une coque percée d'un orifice central 11a et destinée à l'application focalisée du fluide sur la zone cutanée.
La paroi latérale de cette coque est ajourée et son pourtour vient s'encliqueter sur le bord périphérique de la partie basse de la buse 10. L'utilisation du dispositif selon l'invention sera expliquée plus en détail à partir de l'exemple suivant de mise en œuvre du procédé de traitement.

### Exemple de mise en œuvre d'un procédé de traitement cosmétique par cryogénie cyto-sélective

Une étude a été réalisée de manière à valider le dispositif décrit ci-dessus. L'étude a consisté à appliquer un gaz cryogénique 152A sur des sujets présentant des taches brunes sur le dos de la main. L'étude a été réalisée sur 4 sujets, deux hommes et deux femmes, JMPAT, CDEN, AMAH et YPHIL, âgés de 50, 57, 59 et 55 ans. La durée d'application du gaz cryogénique a été réglée à 3 secondes.

Les sujets présentaient les caractéristiques suivantes:
- JMPAT: présence d'une tache brune fortement marquée au niveau de la main droite, au niveau de l'index.
- CDEN: présence d'une tache brune marquée au niveau de la main droite, situé entre l'index et l'auriculaire.
- AMAH: présence de deux taches brunes de taille importante, situées au niveau de la main droite.
- YPHIL: présence de deux taches brunes, presque accolées l'une à l'autre au niveau de la main droite.

Les résultats montrent que le dispositif permet d'éliminer les taches brunes après une seule pulvérisation de 3 secondes par tache. En effet, les taches brunes traitées ont totalement disparu chez tous les sujets, après une période de deux à trois semaines après une seule application. Certains des sujets ayant participé à l'étude avaient déjà été traités par un dermatologue sur d'autres taches brunes similaires du dos de leur main. Le traitement avait consisté en l'application d'une cryothérapie traditionnelle à base d'azote ou de diméthyléther.

Contrairement à ces traitements, l'application superficielle du froid cryogénique au moyen du dispositif de l'invention n'a pas induit chez ces sujets de sensation de douleur, alors qu'une forte douleur avait été ressentie lors du traitement précédent effectué par le dermatologue. De plus, on note l'absence d'inflammation importante et de destruction tissulaire susceptibles de conduire à des marques sous la forme de cicatrices ou d'hypo-pigmentation.

Ces observations confirment que le dispositif produit bien un effet cosmétique cryo-cyto-sélectif, alors que la cryothérapie traditionnelle à base d'azote ou de diméthylether, telle que pratiquée sous contrôle médical en cabinet de dermatologie, n'apporte pas un tel effet. Le dispositif grâce à son action cryo-cyto-sélective ne produit ni la destruction par nécrose de l'ensemble du tissu, ni la destrucution des kératinocytes et donc n'induit pas les phénomènes observés avec la cryothérapie traditionnelle à base d'azote telle que pratiquée en cabinet de dermatologie (inflammation, douleur importante, cicatrice, hypo-pigmentation).

Bien que présentée sur la figure 1 en forme de diffuseur allongé et agencé pour contenir une cartouche de difluoroéthane, la présente divulgation peut s'appliquer à toute forme de diffuseur de fluide cryogénique ayant une température d'ébullition entre - 20°C et - 65°C et une chaleur latente d'évaporation comprise entre 1 et 500 KJ/Kg, muni d'un dispositif de focalisation approprié sur une zone cutanée et d'un système de temporisation préréglé ou réglable.

Comme cela a été décrit ci-dessus, la présente divulgation propose un dispositif pour le traitement cosmétique de tissus cutanés destiné à obtenir une action cryo-cyto-sélective mélanocytes versus kératinocytes. Comme déjà mentionné ci-dessus, on entend par action cryo-cyto-sélective ou cryo-cyto-sélectivité le fait d'agir sélectivement sur une population de cellules d'un tissu considéré sans agir sur au moins une autre ou les autres populations de cellules.

La présente divulgation pourrait s'appliquer à d'autres types de populations de cellules dans d'autres tissus biologiques, le terme population de cellules étant compris dans son sens le plus large, c'est à dire un ensemble de cellules qui présentent les mêmes caractéristiques, par exemple, un type de cellules, une lignée cellulaire, une souche de cellules, procaryotes ou eucaryotes, de toutes origines, humaines, animales ou végétales.

De plus, le dispositif de la présente divulgation peut s'appliquer également au traitement d'éléments constitutifs des cellules c'est-à-dire des organites cellulaires, notamment et de manière non limitative, aux mélanosomes, aux nucléoles, noyau, ribosomes, vésicules, réticulums endoplasmiques, appareils de Golgi, cytosquelette, mitochondries, vacuoles, cytosols, lysosomes, centrosomes et membranes plasmiques.

La présente divulgation telle que décrite plus haut est susceptible de s'appliquer à condition que l'on puisse déterminer une plage de température délétère pour la population de cellules, de structures ou d'organismes cibles et dans laquelle plage une autre population ou le tissu environnant n'est pas impacté de manière notable ou rédhibitoire.

## Revendications

1. Dispositif pour la mise en œuvre d'un traitement cosmétique destiné à faire disparaître des taches brunes cutanées, comprenant un réservoir (2) de fluide cryogénique, une électrovanne (7) permettant le passage du fluide cryogénique vers l'aval, de l'intérieur dudit réservoir vers une buse (10) via un gicleur (9), ladite électrovanne (7) étant associée à un système électronique de temporisation (4) permettant son ouverture pendant une durée prédéterminée, **caractérisé en ce que** ledit gicleur (9) comprend au moins un corps (92) dans lequel est aménagé au moins un conduit interne axial et cylindro-conique formant moyen de limitation du débit de fluide cryogénique.

2. Dispositif selon la revendication 1, **caractérisé en ce que** ledit gicleur est réalisé dans un matériau hydropobe et non conducteur thermiquement.

3. Dispositif selon la revendication 1 ou 2, **caractérisé en ce que** la longueur du conduit interne est comprise entre 3 et 12 mm et son diamètre intérieur entre 0,15 et 3,5 mm.

4. Dispositif selon l'une des revendications précédentes, **caractérisé en ce que** ledit gicleur comprend deux corps (92) assemblés par empilement en écrasant au moins un joint d'étanchéité intercalaire (J1, J2) et de telle sorte que leurs conduits soient raccordés coaxialement en série.

5. Dispositif selon l'une des revendication précédentes, **caractérisé en ce que** ledit corps (92) est pourvu de cavités, respectivement, supérieure (92a) et inférieure (92d) pour le logement de joints (J1, J2) d'étanchéité.

6. Dispositif selon l'une des revendication précédentes, **caractérisé en ce que** ledit corps (92) comprend deux conduits internes, respectivement, un conduit amont (92b) au moins partiellement conique formant chambre de rétention et débouchant en aval dans un conduit cylindrique (92c) de plus faible diamètre.

7. Dispositif selon l'une des revendications précédentes, **caractérisé en ce que** ledit gicleur assure la délivrance du fluide cryogénique avec une pression maximum de 10 bars.

8. Dispositif selon l'une des revendications précédentes, **caractérisé en ce que** ledit système de temporisation permet une durée consécutive d'application du fluide cryogénique de 2 à 10 secondes.

9. Dispositif selon l'une des revendications précédentes, **caractérisé en ce que** ledit gicleur assure la délivrance du fluide cryogénique à une température comprise entre - 4°C et - 15°C.

10. Dispositif selon l'une des revendications précédentes, **caractérisé en ce que** ledit gicleur assure un impact du jet de fluide cryogénique sur une zone de moins de 30 mm².

## Patentansprüche

1. Vorrichtung zur Durchführung einer kosmetischen Behandlung zur Entfernung brauner Hautflecken, mit einem Behälter (2) mit kryogener Flüssigkeit, einem Magnetventil (7), das den Durchgang der kryogenen Flüssigkeit stromabwärts vom Inneren des genannten Behälters über einen Spritzverteiler (9) bis zu einer Sprühdüse (10) ermöglicht, wobei das Magnetventil (7) mit einem elektronischen Zeitsteuerungssystem (4) verbunden ist, das dessen Öffnung für eine vorab festgelegte Zeit ermöglicht, **dadurch gekennzeichnet, dass** der Spritzverteiler (9) mindestens einen Körper (92) umfasst, in dem mindestens ein innerer axialer, zylindrisch-konischer Kanal angeordnet ist, der ein Mittel zur Begrenzung des Durchflusses der kryogenen Flüssigkeit bildet.

2. Vorrichtung nach Anspruch 1, **dadurch gekennzeichnet, dass** der Spritzverteiler aus einem hydrophoben und thermisch nichtleitenden Material hergestellt ist.

3. Vorrichtung nach Anspruch 1 oder 2, **dadurch gekennzeichnet, dass** die Länge des Innenkanals 3 bis 12 mm und sein Innendurchmesser 0,15 bis 3,5 mm beträgt.

4. Vorrichtung nach einem der vorhergehenden Ansprüche, **dadurch gekennzeichnet, dass** der Spritzverteiler zwei Körper umfasst, die durch Stapelung mit mindestens einer dazwischenliegenden Dichtungslage (J1, J2) so zusammengefügt werden, dass ihre Kanäle koaxial in Reihe verbunden sind.

5. Vorrichtung nach einem der vorhergehenden Ansprüche, **dadurch gekennzeichnet, dass** der Körper (92) mit einem oberen (92a) und einem unteren (92d) Hohlraum zur Aufnahme von Dichtungen (J1, J2) versehen ist.

6. Vorrichtung nach einem der vorhergehenden Ansprüche, **dadurch gekennzeichnet, dass** der Körper zwei innere Kanäle umfasst, nämlich einen stromaufwärts gelegenen Kanal (92b), der zumindest teilweise konisch ist und eine Rückhaltekammer bildet und stromabwärts in einen zylindrischen Kanal (92c) mit kleinerem Durchmesser mündet.

7. Vorrichtung nach einem der vorhergehenden Ansprüche, **dadurch gekennzeichnet, dass** der Spritzverteiler die Abgabe der kryogenen Flüssigkeit mit einem maximalen Druck von 10 bar gewährleistet.

8. Vorrichtung nach einem der vorhergehenden Ansprüche, **dadurch gekennzeichnet, dass** das Zeitsteuerungssystem eine aufeinanderfolgende Anwendungsdauer der kryogenen Flüssigkeit von 2 bis 10 Sekunden ermöglicht.

9. Vorrichtung nach einem der vorhergehenden Ansprüche, **dadurch gekennzeichnet, dass** der Spritzverteiler die Abgabe der kryogenen Flüssigkeit bei einer Temperatur zwischen -4°C und - 15°C gewährleistet.

10. Vorrichtung nach einem der vorhergehenden Ansprüche, **dadurch gekennzeichnet, dass** der Spritzverteiler ein Auftreffen des kryogenen Flüssigkeitsstrahls auf eine Zone von weniger als 30 mm2 gewährleistet.

## Claims

1. A device for carrying out a cosmetic treatment intended for eliminating dark spots on the skin, comprising a cryogenic fluid tank (2), a solenoid valve (7) allowing the passage of the cryogenic fluid in the downstream direction, from the inside of said tank to a nozzle (10) via a sprayer (9), said solenoid valve (7) being associated with an electronic timing system (4) enabling it to be opened for a predetermined time, **characterised in that** said sprayer (9) comprises at least one body (92) in which at least one internal axial and cylindro-conical conduit forming means for limiting the flow of cryogenic fluid is arranged.

2. A device according to claim 1, **characterized in that** said sprayer is made of a hydrophobic and thermally non-conductive material.

3. A device according to claim 1 or 2, **characterized in that** the length of the internal conduit is between 3 and 12mm and its inner diameter is between 0.15 and 3.5mm.

4. A device according to one of the preceding claims, **characterized in that** said sprayer comprises two bodies stacked into an assembly by crushing at least one intermediate seal (J1, J2) and so that their conduits are coaxially connected in series.

5. A device according to one of the preceding claims, **characterized in that** said body (92) is provided with upper (92a) and lower (92d) cavities, respectively, for housing seals (J1, J2).

6. A device according to one of the preceding claims, **characterized in that** said body comprises two internal conduits, respectively, an at least partially conical upstream conduit (92b) forming a retention chamber and opening downstream into a cylindrical conduit (92c) having a smaller diameter.

7. A device according to one of the preceding claims, **characterized in that** said sprayer ensures the delivery of the cryogenic fluid with a maximum pressure of 10 bars.

8. A device according to one of the preceding claims, **characterized in that** said timing system allows a consecutive duration of application of the cryogenic fluid of 2 to 10 seconds.

9. A device according to one of the preceding claims, **characterized in that** said sprayer ensures the delivery of the cryogenic fluid at a temperature between -4°C and -15°C.

10. A device according to one of the preceding claims, **characterized in that** said sprayer ensures an impact of the cryogenic fluid jet on an area of less than 30mm2.
